# EUROPEAN PATENT APPLICATION

(11) **EP 3 178 506 A1**
(43) Date of publication of application: **14.06.2017**
(21) Application number: 15199669.1
(22) Date of filing: 11.12.2015
(51) Int. Cl.: A61M 5/172, A61M 39/02, A61M 5/142

(54) **FLUID INTERFACE DEVICE FOR DELIVERING FLUID TO AND/OR WITHDRAWING FLUID FROM A PA-TIENT**

(71) Applicant: SeraIP AG, 6370 Stans (CH)
(72) Inventor: The designation of the inventor has not yet been filed
(74) Representative: Schmauder & Partner AG Patent- & Markenanwälte VSP

(57) **Abstract**

A fluid interface device for delivering fluid to and/or withdrawing fluid from a patient, the device comprises a peripheral base element (2) and a fluid transmission element (4) sealingly connected to the base element and forming a central portion of the device. The fluid transmission element comprises a front platelet (6) with a primary face (8) and a secondary face (10) opposed thereto, the primary face being in contact with a patient's body fluid region (12) when the device is implanted in the patient, the fluid transmission element further comprising a counterplate (14) sealingly stacked against the secondary face of the front platelet and forming a buffer volume (16) therebetween. The front platelet comprises at least one array of microchannels (18) defining a fluid passage between the buffer volume and the primary face, the microchannels having an opening of 0.2 to 10 µm. The counterplate has at least two fluid ports (20a; 20b) for fluid delivery to and/or fluid withdrawal from the buffer volume.

## Description

### Field of the Invention

The present invention generally relates to a fluid interface device for delivering fluid to and/or withdrawing fluid from a patient. Moreover, the invention relates to a system for delivering fluid to and/or withdrawing fluid from a patient. Furthermore, the invention relates to a method of operating the system.

### Background of the Invention

According to the World Health Organization, in 2014 the global prevalence of diabetes was estimated to be 9% among adults aged 18 or more (Global status report on noncommunicable diseases 2014. Geneva, World Health Organization, 2012). Treatment of diabetes involves lowering blood glucose and the levels of other known risk factors that damage blood vessels. For patients with type 1 diabetes, but also for patients with progressed forms of type 2 diabetes, the necessary interventions include administration of insulin. Because of inevitable variations in external influencing factors and often also because of a lack of discipline, the glucose levels in blood often fluctuate substantially, which can lead to a number of complications of the vascular and nervous systems. For such patients, insulin pumps have gained increasing popularity. Most of these pumps emit insulin continuously at a low-dosage basal rate which can be increased on demand, notably before meals. In order to optimize use of an insulin pump, it is highly desirable to also have a system for continuous or periodic monitoring of the blood glucose level.

US 4,704,029 discloses a blood glucose monitor system for use as an implant for controlling an insulin pump, or as a portable device for use by a diabetic for home blood glucose monitoring. The glucose monitor measures the glucose level of blood by utilizing a refractometer which measures the index of refraction of blood adjacent to an interface with a transparent surface of the refractometer, by directing light at the interface to measure the index of refraction of the blood by the amount of radiation reflected by the interface, particularly light incident near the critical angle. A proposed device designed to directly contact the blood comprises an optical interface made of a contacting material with a critical surface tension selected so as to minimize deterioration by antibodies and proteins contained in the blood. One such exemplary material is dichloro-dimethyl-silane, also known as G.E. Dry Film.

Further blood glucose monitoring systems have been proposed which are based on obtaining a capillary blood sample from a patient; see for example, WO 2009/071775 A1. Such system usually comprise either an agglomerating agent or some kind of fibrous filter for removing blood cells and other particles contained in the blood in order to carry out the glucose measurement on blood plasma. As will be known, the term "blood plasma" denotes the liquid fraction obtained by removing the blood cells and other particles contained in blood.

However, an efficient and reliable blood glucose monitor system for controlling an insulin delivery system should preferably be implantable so as to be able to continuously or periodically sample blood plasma from a venous - or possibly arterial - blood vessel. In spite of many attempts, this task does not seem to have been satisfactorily solved. The apparently obvious approach of implanting some kind of microporous membrane into a blood vessel wall, which would allow extracting blood plasma and keep blood cells and other particles in the blood vessel, faces the problem of very rapid obstruction of the micropores by clot formation.

Accordingly, it would be highly desirable to provide a fluid interface for withdrawing blood plasma from a patient in a continuous, reliable manner over extended periods of time, preferably for several years or even longer. It would be equally desirable to have such a fluid interface for withdrawing other types of a patient's fluids such as cerebrospinal fluid. Moreover, it would also be desirable to use the same fluid interface for delivering appropriate fluids such as therapeutic or prophylactic agents to a patient's bloodstream.

### Summary of the Invention

It is thus an object of the present invention to provide an improved fluid interface device for delivering fluid to and/or withdrawing fluid from a patient. In particular, such a device shall overcome the limitations and disadvantages of presently known devices.

According to one aspect of the invention, there is provided a fluid interface device for delivering fluid to and/or withdrawing fluid from a patient, the device comprising:
- a peripheral base element;
- a fluid transmission element sealingly connected to the base element and forming a central portion of the device, the fluid transmission element comprising a front platelet with a primary face and a secondary face opposed thereto, the primary face being in contact with a patient's body fluid region when the device is implanted in the patient, the fluid transmission element further comprising a counterplate sealingly stacked against the secondary face of the front platelet and forming a buffer volume therebetween; the front platelet comprising at least one array of microchannels defining a fluid passage between the buffer volume and the primary face, the microchannels having an opening of 0.2 to 10 µm;
- the counterplate having at least two fluid ports for fluid delivery to and/or fluid withdrawal from the buffer volume.

The core part of the device is constituted by the fluid transmission element, which is formed as a chamber containing a buffer volume. Specifically, the transmission element is configured in a sandwich-like manner with a front platelet having a primary face oriented towards the patient's body fluid region of interest. The front platelet comprises at least one array of microchannels defining a fluid passage between the buffer volume and the primary face. The chamber further comprises a counterplate sealingly stacked against the secondary face of the front platelet having at least two fluid ports for fluid delivery to and/or fluid withdrawal from the buffer volume.

The device of the present invention is generally intended for a achieving a reliable, well defined and uninterrupted transfer of predetermined fluids from or into a patient's body fluid region. Such transfer includes but is not limited to withdrawing blood plasma or cerebrospinal fluid and to delivery of therapeutic or prophylactic agents to various body regions of a patient in need thereof. A highly important task of the device is to achieve a filtering function, i.e. to prevent the passage of cells and other particles through the fluid passage.

As will be described in more detail further below, the optimum size of the microchannels will depend on the particular application. In general it will be selected in the range of 0.2 to 10 µm. The lower limit is primarily determined by the available forming technology, but also by the need to have sufficient throughput. The upper limit is determined by the size of particles that should be prevented from entering into the microchannels. For applications involving the withdrawal of blood plasma the microchannels should have an opening in the range of 0.9 to 2.2 µm, most typically of around 1.6 µm. The term "opening" shall be understood as the diameter in the case of microchannels with circular cross section; for non-circular microchannels the term "opening" shall be understood as the smallest transversal size of the cross section. Currently available technologies for forming openings with the above mentioned diameter range usually require a height to diameter ratio ("aspect ratio") of up to 5. In other words, the thickness of the front platelet in the region surrounding the microchannels needs to be small enough, i.e. in the range of 1 to 50 µm depending on the microchannel diameter. In order to provide sufficient stiffness of the front platelet, reinforcing regions with a substantially higher thickness are provided at locations displaced from the microchannels.

The device of this invention further includes some type of peripheral base element circumferentially arranged around the fluid transmission element. The size, shape and material of the peripheral base element are selected in accordance with the application. In most cases it is primarily intended for reliably fixing the device to a structure of the patient's body. This will be discussed in detail further below.

Advantageous embodiments are defined in the dependent claims and are described below.

Advantageously, the front platelet is made of material that is suitable to a photolithographic processing, which is a very convenient technique for forming narrow structures with a well-defined shape. The counterplate should be made of a material that is compatible with that of the front platelet and that has advantageous properties in view of any fluid connections to be attached thereto. Therefore, according to an advantageous embodiment (claim 2), the front platelet is made of silicon (Si) and/or silicon nitride (Si₃N₄) and the counterplate is made of glass. Suitable sandwich structures made of Si and Si₃N₄ layers are generally known in the field of microtechnology. In some embodiments the front platelet is functionalized, i.e. provided with a suitable coating. The type and thickness of such coating will depend on the particular application. For the sampling of blood plasma there are known functionalizations aiming at the prevention of clot formation and coagulation.

According to an advantageous embodiment (claim 3), the front platelet and the counterplate are joined to each other by anodic bonding. In particular, this method allows formation of strong and medium-tight connections between Si and glass structures.

Various shapes of the fluid transmission element are feasible in principle. In a particularly advantageous embodiment (claim 4), the counterplate is substantially planar and the buffer volume is enclosed within a peripheral protrusion zone of the secondary face of the front platelet. This configuration is favored by the fact that the front plate needs to be processed anyway in order to produce the microchannels; particularly in the case of Si parts there are established methods for forming elevated or recessed regions. As to what concerns the counterplate, which is preferably made of glass, it is generally convenient to use flat or planar shapes.

For many applications it is advantageous if the buffer volume comprises at least two separate compartments, each one being in connection with a respective microchannels array and each being provided with at least two fluid ports (claim 5). This embodiment effectively incorporates two or possibly more than two independently operable subunits of the fluid interface, whereby it is possible to use each subunit for a separate task. In particular, one could use one subunit for sampling purposes, i.e. for withdrawing an amount of fluid such as blood plasma or cerebrospinal fluid from the patient, whereas another subunit could be used for delivery purposes, i.e. for introducing a therapeutic or prophylactic agent to the patient. However, it should be understood that both subunits will generally need to allow for withdrawal and for delivery of fluids in order to perform subsidiary tasks such as flushing.

According to an advantageous embodiment, the fluid interface device further comprises a spacer element with a first spacer face that is adhesively connected to an external face of the counterplate, the spacer element comprising traversing channels connecting the first spacer face and a second spacer face, each traversing channel being arranged to form a passage between one of the counterplate's fluid ports and a corresponding channel opening at the second spacer face (claim 6). Such a spacer element opens various possibilities for establishing a connection between the fluid transmission element, which is located very close to a body region and will generally be small and sensitive, and an external unit used to drive and supply the device. Although the spacer element could be made of a variety of compatible materials, it is advantageously made of a thermoplastic polymer, particularly a non-fluorinated thermoplastic polymer that is suitable for connection by means of an adhesive.

Advantageously, the fluid interface device further comprises a fluid supply connector and means for releasably attaching the fluid supply connector to the spacer element, the fluid supply connector comprising connector channels each leading from a lateral entrance port to an exit port coinciding with a channel opening at the second spacer face when the fluid supply connector is attached to the spacer element (claim 7). Such an embodiment is particularly useful for a device intended for implantation in a patient's blood vessel wall. Firstly, such a configuration allows carrying out the implantation of the comparatively compact device without being hindered by any supply tubing connecting the device with an extracorporeal unit, as the latter can be connected after implantation. Secondly, in case of an infection in the supply tubing system, e.g. in a region where such tubing is conducted through the skin, it would be possible to continue using the implanted fluid interface device and merely install a new supply system.

For construction purposes it is advantageous if each one of the connector channels is formed as a pair of grooves in adjacent faces of mutually contacted connector parts (claim 8). Such a design allows comparatively simple production of curved or bent channels in a solid piece.

According to an advantageous embodiment, the fluid transmission element and the base element are sealingly connected to each other by a ridge structure surrounding the fluid transmission element, the ridge structure being made of a biocompatible thermoplastic formed around the fluid transmission element by injection molding (claim 9). In the present context, a biocompatible material shall be understood as a material that is non-toxic and does not have any other undesirable properties such as allergenicity towards the intended patient.

In a first, inner contacting region of the ridge structure a medium tight closure is formed against an outer circumferential surface of the fluid transmission element as a result of the injection molding process. For this purpose, it is advantageous if the fluid transmission element is provided with a structured peripheral region. In particular, the front platelet may be formed with a radially protruding section provided with holes, rims or undercuts which will be filled up with injected thermoplast and thereby will strongly improve the connection between the ridge structure and the fluid transmission element.

In a second, outer contacting region of the ridge structure, a medium tight connection is formed with the peripheral base element by means of a suitable contacting method. The best choice of such contacting method will depend on the materials to be joined. According to an advantageous embodiment, the peripheral base element is sealingly connected to the ridge structure by injection molding thereon a covering part; according to another advantageous embodiment, the peripheral base element is sealingly connected to the ridge structure by ultrasonic welding (claim 10). As will be known to the skilled person, the use of a welding technique requires that the parts to be joined are made of compatible materials, preferably of the same material, which in the present case means a compatible or identical biocompatible thermoplastic polymer.

An important application of the fluid interface device is for implantation in a patient's blood vessel wall, particularly in a venous wall. Advantageously, the device is implanted in a wall section of a patient's upper arm vein. Therefore, according to an advantageous embodiment (claim 11) the peripheral base element is formed as a foamed pad of a thermoplastic fluoropolymer which is suitable for implantation in a patient's blood vessel wall, and the injection molded ridge structure is formed as a non-foamed body of said thermoplastic fluoropolymer. The implantation of various types of pads into venous walls has been extensively studied and thus is basically known. Accordingly, there exist well tested materials for this purpose, among which foamed thermoplastic fluoropolymers have turned out to be very suitable. In order to connect the foamed pad to the ridge structure, which is preferably done by ultrasonic welding, it is advantageous if the injection molded ridge structure is made of the same thermoplastic fluoropolymer as the foamy peripheral pad. By the above definitions, both parts are made of biocompatible materials. Implanting the fluid interface device in a patient's venous wall is a promising approach for establishing a reliable, uninterrupted sampling of the patient's venous blood and for regulated delivery of any suitable therapeutic or prophylactic agent. An important application field is for diabetic patients.

Another application field of the fluid interface requires fixation to an osseous structure of the patient, e.g. to a skull section. Therefore, according to another advantageous embodiment (claim 12) the peripheral base element is formed as rigid frame structure suitable for fixation to an osseous structure of a patient. Appropriate fixation means are known, e.g. for implantation of hearing aids. In the present context, the fluid interface device could be applied to a patient's skull for sampling intracranial fluid and/or for delivering thereto any suitable therapeutic or prophylactic agent thereby allowing to avoid the blood-brain-barrier. For this mode of use the microchannels of the device will serve to prevent leucocytes and antibodies of the intracranial fluid from clogging the microchannel array and/or entering into the buffer volume.

A further application field of the fluid interface relates to subcutaneous or possibly intramuscular placement which does not require firm fixation to a body part. Accordingly, in a further embodiment (claim 13) the fluid interface device further comprises a fluid supply connector attached to the spacer element, the fluid supply connector comprising connector channels each leading from an entrance port to an exit port coinciding with a channel opening at the second spacer face, the fluid supply connector being formed as a sealing mass that encapsulates the spacer element and the fluid transmission element and that furthermore forms the peripheral base element. In such embodiments a certain degree of fixation will be provided by a transcutaneous passage connecting the fluid interface device to an external control and supply device.

According to another aspect of the invention (claim 14), there is provided a system for delivering fluid to and/or withdrawing fluid from a patient's body region.

The system comprises a fluid interface device as defined above and furthermore comprises fluid storage means and fluid transfer means for controlled fluid delivery to and fluid withdrawal from the buffer volume via the fluid ports. The system is configured to perform at least the following steps according to a pre-defined step sequence:
a) running flushing medium through the buffer volume;
b) running flushing medium through the microchannels array;
c) withdrawing patient's body fluid through the microchannels array; and
d) delivering a therapeutic agent to the patient.

According to a further aspect of the invention (claim 15), there is provided a method of operating the system defined above, in which method a flushing medium is delivered to the buffer volume so as to maintain an overpressure relative to a base pressure in the patient's body region when the system is not withdrawing patient's body fluid or delivering a therapeutic agent to the patient, thereby preventing any flow from the patient's body region through the microchannels into the buffer volume.

The flushing medium typically will be a 30% / 70% ethanol / water solution containing a known anticoagulant such as heparin or aspirin. By diffusing from the buffer medium through the microchannels, the anticoagulant will contribute to avoid undesirable blood clot formation in or near the microchannel array.

In principle one could establish a small but constant flow of flushing medium from the buffer volume into the patient's body region. However, this would require excessive amounts of flushing medium and is generally not desirable. In many practical situations it will be sufficient to simply keep the buffer volume under a small overpressure relative to a base pressure in the patient's body region. To do so, one can periodically feed a small portion of buffer medium into the buffer volume, for example 10 µl every 5 min.

The above defined system and operating method may be termed "liquid membrane management" in the sense that an undesirable clogging of the microchannels, but also an undesirable entry of certain cells and other particles into the buffer volume is avoided by maintaining a layer of protecting liquid in the microchannels at all times.

### Brief description of the drawings

The above mentioned and other features and objects of this invention and the manner of achieving them will become more apparent and this invention itself will be better understood by reference to the following description of various embodiments of this invention taken in conjunction with the accompanying drawings, wherein:
- Fig. 1: shows a first embodiment of a fluid interface device, in a sec-tional view;
- Fig. 2: shows a part of the fluid interface device of Fig. 1 with an at-tached spacer element, in a sectional view;
- Fig. 3: shows a second embodiment of a fluid interface device, in a schematic sectional view;
- Fig. 4: shows a front platelet of a third embodiment, in a plan view seen from the secondary face;
- Fig. 5: shows the front platelet of Fig. 4 in a perspective view;
- Fig. 6: shows the front platelet of Fig. 4 in a sectional view according to section F-F;
- Fig. 7: shows an enlarged portion G of Fig. 6;
- Fig. 8: shows the front platelet of Fig. 4 with a counterplate attached thereto, in a plan view seen from the secondary face;
- Fig. 9: shows the front platelet and counterplate of Fig. 8 in a sectional view according to section B-B;
- Fig. 10: shows the front platelet and counterplate of Fig. 8 in a sectional view according to section A-A;
- Fig. 11: shows the front platelet and counterplate of Fig. 8 in a perspec-tive view;
- Fig. 12: shows a fluid interface device including the front platelet and counterplate of Fig. 8 and a fluid supply connector attached thereto, in a plan view seen from the primary face;
- Fig. 13: shows the arrangement of Fig. 12 in a sectional view according to section A-A;
- Fig. 14: shows the arrangement of Fig. 12 in a sectional view according to section B-B;
- Fig. 15: shows the arrangement of Fig. 12 in a perspective view;
- Fig. 16: shows the arrangement of Fig. 12 in a plan view seen from the secondary face;
- Fig. 17: shows the arrangement of Fig. 16 in a sectional view according to section C-C;
- Fig. 18: shows the arrangement of Fig. 16 in a sectional view according to section D-D;
- Fig. 19: shows another fluid interface device including the front platelet and counterplate of Fig. 8 and a fluid supply connector attached thereto, in a plan view seen from the side opposite to the primary face;
- Fig. 20: shows the arrangement of Fig. 19 in a sectional view according to section A-A;
- Fig. 21: shows the arrangement of Fig. 19 in a sectional view according to section B-B; and
- Fig. 22: shows the arrangement of Fig. 19 in a perspective view.

### Detailed description of the invention

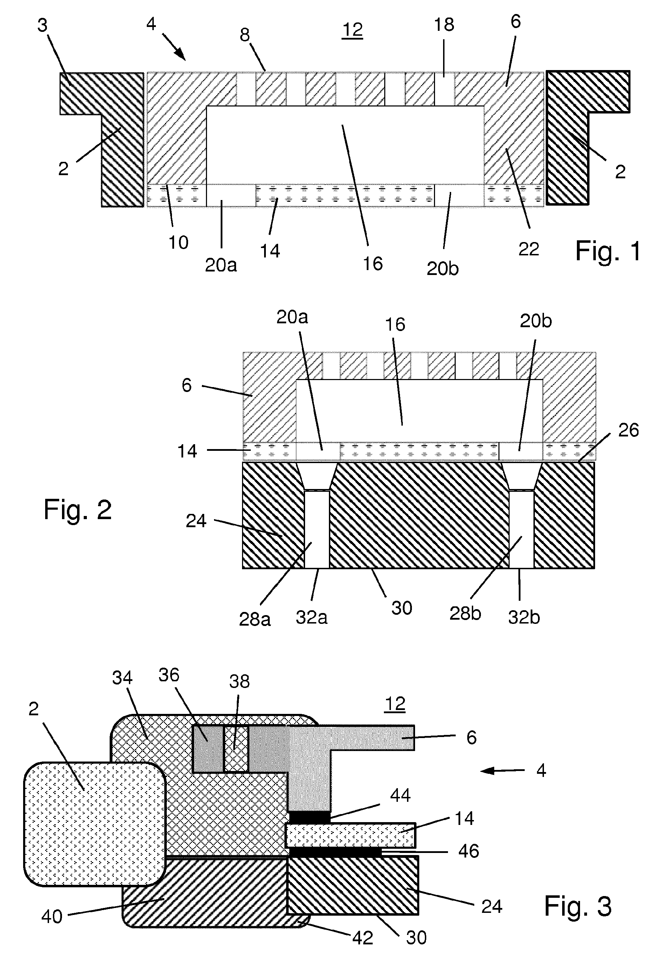

In order to better explain general principle of the present invention, Figs. 1 and 2 show a basic embodiment of a fluid interface device, in a schematic representation and not to scale. The device comprises a peripheral base element 2, which in the example shown is configured as a simple cylindrical piece with a radially protruding flange 3. A composite part generally denoted as fluid transmission element 4 is sealingly connected to the base element 2 and forms a central portion of the device. The base element 2 is generally intended to provide some kind of attachment or fixture to a patient's body part and could be integrally formed with the fluid transmission element. The latter comprises a front platelet 6 with a primary face 8 and with a secondary face 10 opposed thereto. The primary face is in contact with a patient's body fluid region generally denoted as 12 when the device is implanted in the patient. The fluid transmission element further comprising a counterplate 14 that is sealingly stacked against the secondary face of the front platelet and forms a buffer volume 16 therebetween. Importantly, the front platelet 6 comprises at least one array of microchannels 18 defining a fluid passage between the buffer volume and the primary face. Depending on the intended use of the device, the microchannels are formed with an opening of 0.2 to 10 µm. The counterplate has two fluid ports 20a; 20b for fluid delivery to and/or fluid withdrawal from the buffer volume.

In the example shown, the counterplate 10 is substantially planar and is made of glass. In contrast, the front platelet 6 has a peripheral protrusion zone 22 directed towards the counterplate 10 and forming a lateral wall enclosing the buffer volume 16. The front platelet 6 is made of Si and/or Si₃N₄ and is joined to the counterplate 14 by anodic bonding.

Fig 2. shows the fluid interface device of Fig. 1, but without peripheral base element 2. A spacer element 24 made of a thermoplastic polymer has a first spacer face 26 that is connected to an external face of the counterplate 14 by means of a suitable adhesive. The spacer element 24 comprises traversing channels 28a; 28b connecting the first spacer face and a second spacer face 30 opposed therefrom. Each traversing channel is arranged to form a passage between one of the counterplate's fluid ports 20a, 20b and a corresponding channel opening 32a 32b at the second spacer face. As will be seen from forthcoming examples, the spacer element 24 is a useful means for coupling with an appropriately configured tubing connector for supply and delivery of fluid from and to an external device.

The basic structure of a fluid interface device suitable for implantation in a venous wall is further illustrated in Fig. 3. For this purpose the peripheral base element 2 is formed as a foamy pad of a thermoplastic fluoropolymer which is suitable for implantation in a patient's blood vessel wall. Such a materials are commercially available, e.g. as GORE® ACUSEAL Cardiovascular Patch. In order to form a compact, reliable and medium tight connection between the foamy pad 2 and the fluid interface structure 4, an arrangement as shown in Fig. 3 can be used. Such arrangement comprises a ridge structure 34 surrounding the fluid transmission element 4 and sealingly connecting the latter with the base element 2. The ridge structure 34 is made of a biocompatible thermoplastic fluoropolymer formed around the fluid transmission element 4 by injection molding. In order to promote a good adhesion of the ridge structure 34 with the fluid transmission element 4, the front platelet 6 has an outwardly protruding collar 36 provided with a plurality of holes 38. As shown in Fig. 3, the injection molded material of the ridge structure 34 is disposed around the collar 36 and within the holes 38, which provides a form-locking effect. It will be understood that instead of holes the collar could be provided with other types of locking structures such as recesses and protrusions.

In the example shown in Fig. 3, a covering part 40, which is generally ring-like and made of the same thermoplastic fluoropolymer as the ridge structure 34 surrounds the spacer element 24 and is in contact with the ridge structure 34. The ridge structure 34 and the covering part 40 surround a portion of the peripheral base element 2 in a C-type manner. The C-shaped boundary zone between the peripheral base element 2, the ridge structure 34 and the covering part 40 is connected by ultrasonic welding. It will be understood that this convenient joining method requires that the thermoplastic fluoropolymer of the ridge structure and of the covering part is either the same as or is compatible with the fluoropolymer forming the foamy pad peripheral base element 2. In the example shown, the covering part 40 is formed with an overlap zone 42 extending over the second face 30 of the spacer element 24.

As also shown in the schematic representation of Fig. 3, the front platelet 6 and the counterplate 14 are joined to each other in a first contacting zone 44 formed by anodic bonding. Further, the counterplate 14 and the spacer 24 are joined to each other in a second contacting zone 46 by means of a suitable adhesive.

A convenient manner of assembling the exemplary device of Fig. 3 may be summarized as follows:
- place a previously assembled fluid transmission element 4 onto a corresponding holder with the front platelet 6 downwards
- form the peripheral ridge 34 by injection molding around the fluid transmission element 4
- stack the spacer element 24 onto the counterplate 14 and join with suitable adhesive
- place a suitably formed peripheral base element 2 made of foamy thermoplastic fluoropolymer on top of the peripheral ridge 34
- separately form the cover part 40 and stack the same on top of the peripheral ridge 34 and spacer element 24
- connect the cover part 40, the peripheral ridge 34 and the peripheral base element 2 sandwiched therebetween by means of ultrasonic welding.

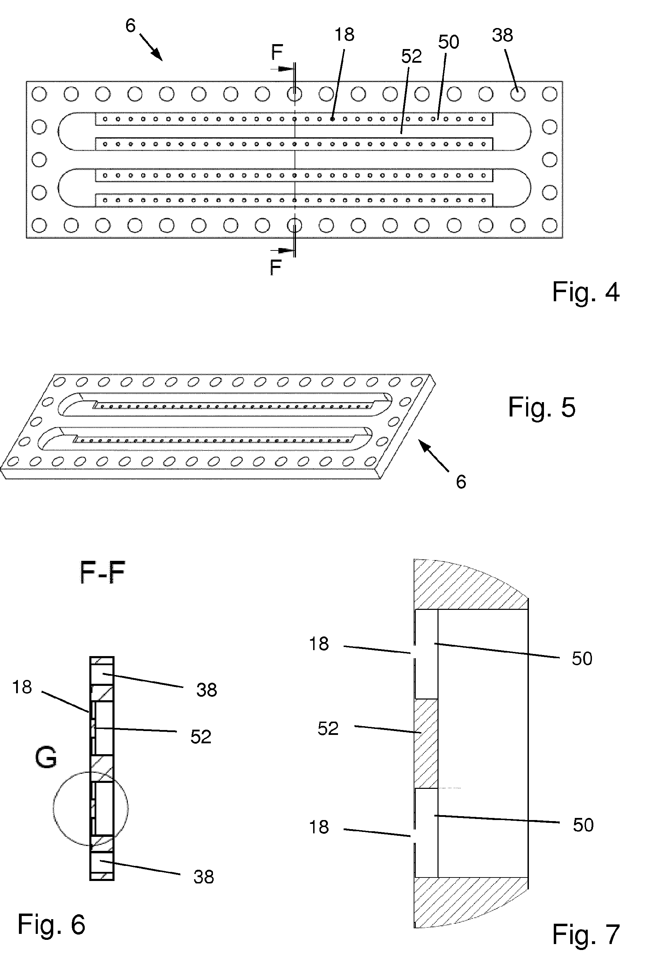

Further details of an embodiment of the fluid interface device suitable for implantation in a blood vessel are illustrated in Figs. 4 to 18. Features corresponding to those in the embodiments explained above are generally denoted with the same reference numerals as above.

As will be seen from Figs. 4 to 7, the microchannels 18 are positioned in elongated grooves 50 formed in the front platelet 6. The grooves 50 constitute a part of the front platelet having minimal thickness so as to allow formation of the narrow microchannels 18. Also shown in Figs. 4 to 7 are the holes 38 formed in the collar zone 36. A thicker platelet zone 52 located between a pair of grooves 50 serves as mechanical reinforcement.

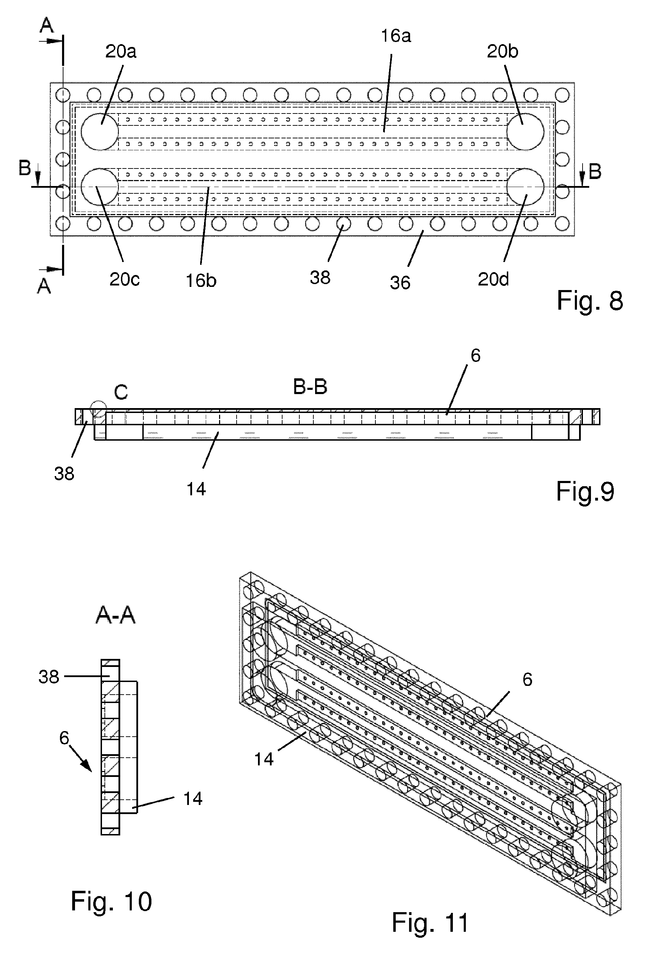

As evident from Figs. 8 to 11, a counterplate 14 stacked on the secondary side of the front platelet 6 leaves free the collar zone 36. In the example shown there are two separate buffer volumes 16a and 16b, each of which is provided with a pair of fluid ports 20a, 20b or 20c, 20d, respectively.

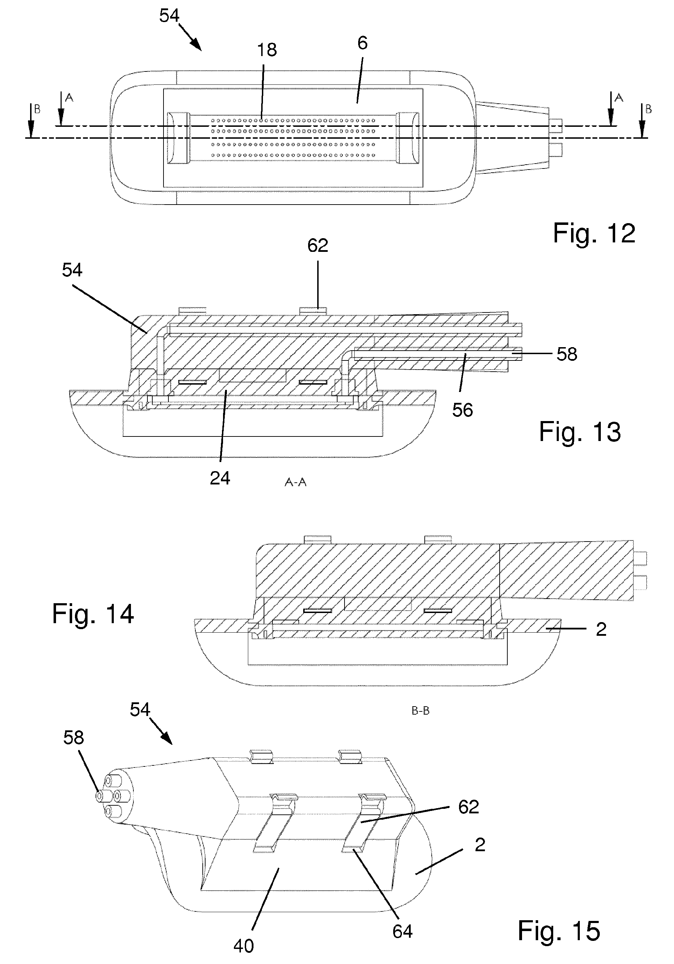

Figs. 12 to 18 show a fluid interface with a fluid supply connector 54 attached thereto. In particular, Fig. 12 shows connector channels 56 each leading from a lateral entrance port 58 to an exit port 60 coinciding with a channel opening 32 at the second spacer face 30. As shown in Figs. 15, 16 and 18, the arrangement has four hook-like elements 62 for releasably attaching the fluid supply connector 54 to the spacer element 24. In the example shown, the hook-like elements 62 traverse suitable openings 64 provided in the covering part 40 placed on top and around spacer 24.

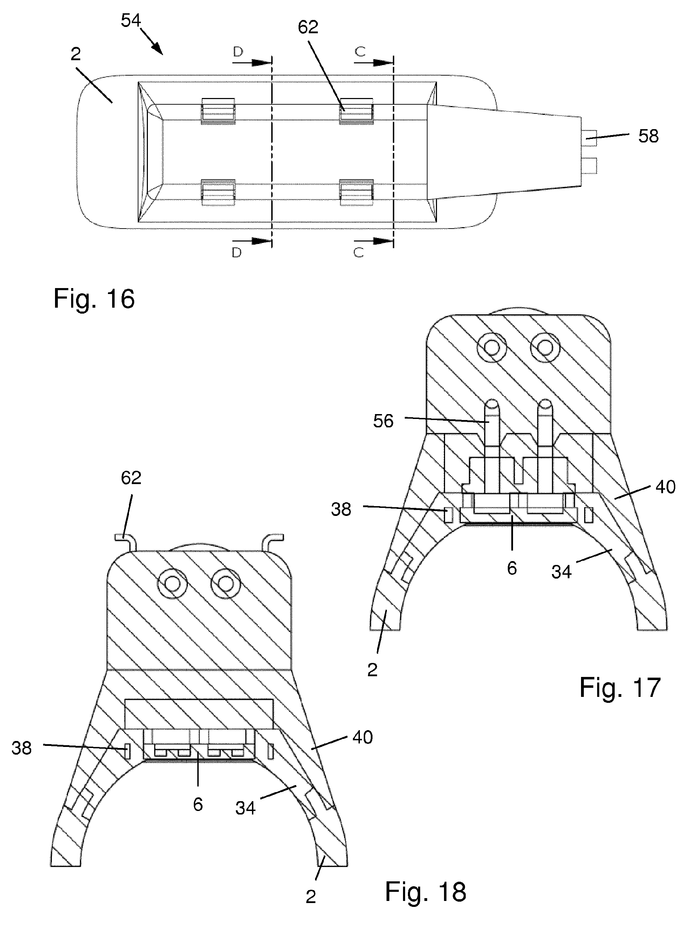

As illustrated in Fig. 17 and 18, the ridge structure 34 and the peripheral base element 2 are formed having a concave cross section dimensioned in accordance with the cross section of a blood vessel into which the entire device can be implanted.

Fig. 18 furthermore shows an advantageous manner of sealingly connecting the peripheral base element 2 and the ridge structure 34 by injection molding thereon thereon the covering part 40, whereby a medium-tight closure is formed between parts 40 and 34 and also with part 2 inserted therebetween.

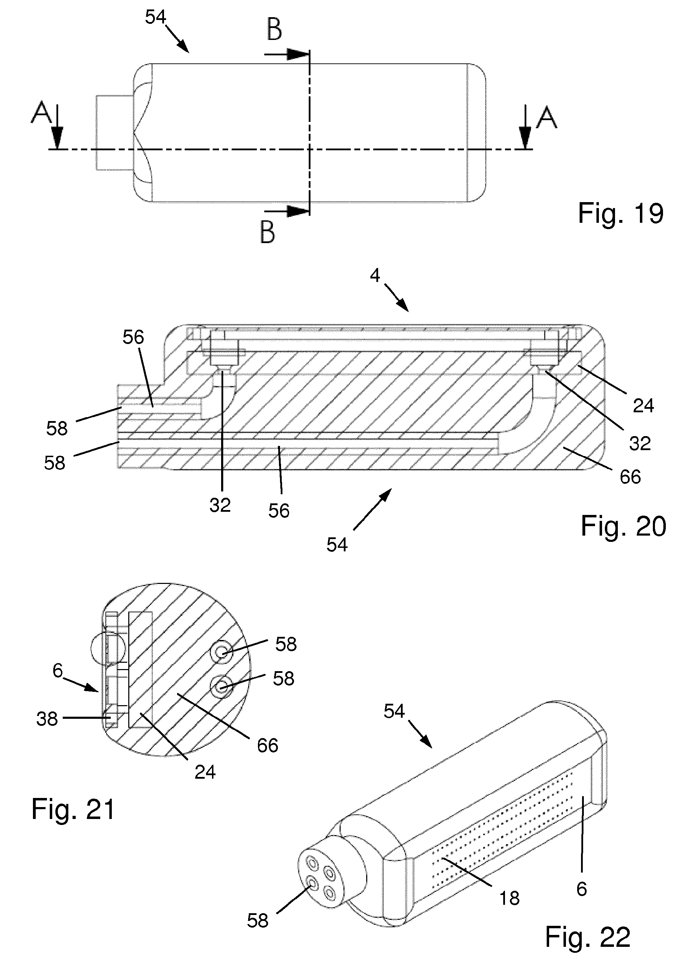

A further embodiment suitable for subcutaneous or intramuscular placement is shown in Figs. 19 to 22. A fluid transmission element generally denoted as 4 is provided with a spacer element 24 in the manner as described further above. Connector channels 56 are provided to form a fluid connection between entrance ports 58 and corresponding spacer channel openings 32. The fluid supply connector (54) is configured as a sealing mass which forms an encapsulation of the spacer element 24 and at the same time forms the peripheral base element 2.

## Claims

1. A fluid interface device for delivering fluid to and/or withdrawing fluid from a patient, the device comprising:
- a peripheral base element (2);
- a fluid transmission element (4) sealingly connected to the base element and forming a central portion of the device, the fluid transmission element comprising a front platelet (6) with a primary face (8) and a secondary face (10) opposed thereto, the primary face being in contact with a patient's body fluid region (12) when the device is implanted in the patient, the fluid transmission element further comprising a counterplate (14) sealingly stacked against the secondary face of the front platelet and forming a buffer volume (16) therebetween; the front platelet comprising at least one array of microchannels (18) defining a fluid passage between the buffer volume and the primary face, the microchannels having an opening of 0.2 to 10 µm;
- the counterplate having at least two fluid ports (20a; 20b) for fluid delivery to and/or fluid withdrawal from the buffer volume.

2. The fluid interface device according to claim 1, wherein the front platelet is made of Si and/or Si₃N₄, and wherein the counterplate is made of glass.

3. The fluid interface device according to claim 1 or 2, wherein the front platelet and the counterplate are joined to each other by anodic bonding.

4. The fluid interface device according to one of claims 1 to 3, wherein the counterplate (10) is substantially planar and wherein the buffer volume (16) is enclosed within a peripheral protrusion zone (22) of the secondary face of the front platelet (6).

5. The fluid interface device according to one of claims 1 to 4, wherein the buffer volume comprises at least two separate compartments, each one being in connection with a respective microchannels array and fluid ports.

6. The fluid interface device according to one of claims 1 to 5, further comprising a spacer element (24) preferably made of a thermoplastic polymer, the spacer element (24) having a first spacer face (26) that is adhesively connected to an external face of the counterplate (14), the spacer element comprising traversing channels (28a; 28b) connecting the first spacer face and a second spacer face (30), each traversing channel being arranged to form a passage between one of the counterplate's fluid ports (20a; 20b) and a corresponding channel opening (32a; 32b) at the second spacer face.

7. The fluid interface device according to claim 6, further comprising a fluid supply connector and means for releasably attaching the fluid supply connector to the spacer element (24), the fluid supply connector comprising connector channels each leading from a lateral entrance port to an exit port coinciding with a channel opening (32a; 32b) at the second spacer face (30) when the fluid supply connector is attached to the spacer element (24).

8. The fluid interface device according to claim 7, wherein each one of said connector channels is formed as a pair of grooves in adjacent faces of mutually contacted connector parts.

9. The fluid interface device according to one of claims 1 to 8, wherein the fluid transmission element (4) and the base element (2) are sealingly connected to each other by means of a ridge structure (34) surrounding the fluid transmission element (4), the ridge structure being made of a biocompatible thermoplastic formed around the fluid transmission element (4) by injection molding.

10. The fluid interface device according to claim 9, wherein the peripheral base element (2) is sealingly connected to the ridge structure (34) by injection molding thereon a covering part (40) or by ultrasonic welding.

11. The fluid interface device according to claim 9 or 10, wherein the peripheral base element (2) is formed as a foamed pad of a thermoplastic fluoropolymer which is suitable for implantation in a patient's blood vessel wall, and wherein the injection molded ridge structure (34) is formed as a non-foamed body of said thermoplastic fluoropolymer.

12. The fluid interface device according to one of claims 1 to 10, wherein the peripheral base element (2) is formed as rigid frame structure suitable for fixation to an osseous structure of a patient.

13. The fluid interface device according to claim 6, further comprising a fluid supply connector (54) attached to the spacer element (24), the fluid supply connector comprising connector channels each leading from an entrance port to an exit port coinciding with a channel opening (32a; 32b) at the second spacer face (30), the fluid supply connector (54) being configured as a sealing mass (66) that encapsulates the spacer element (24) and the fluid transmission element (4) and furthermore forms the peripheral base element (2).

14. A system for delivering fluid to and/or withdrawing fluid from a patient's body region, the system comprising a fluid interface device according to one of claims 1 to 12, fluid storage means and fluid transfer means for controlled fluid delivery to and fluid withdrawal from the buffer volume via the fluid ports (20a, 20b), the system being configured to perform at least the following steps according to a pre-defined step sequence:
a) running flushing medium through the buffer volume;
b) running flushing medium through the microchannels array;
c) withdrawing patient's body fluid through the microchannels array; and
d) delivering a therapeutic agent to the patient.

15. A method of operating the system according to claim 14, in which method a flushing medium is delivered to the buffer volume so as to maintain an overpressure relative to a base pressure in the patient's body region when the system is not withdrawing patient's body fluid or delivering a therapeutic agent to the patient, thereby preventing any flow from the patient's body region through the microchannels into the buffer volume.
